# EUROPÄISCHE PATENTSCHRIFT

(11) **EP 0 337 189 B1**
(45) Veröffentlichungstag und Bekanntmachung des Hinweises auf die Patenterteilung: **30.06.1993**
(21) Anmeldenummer: 89105556.8
(22) Anmeldetag: 29.03.1989
(51) Int. Cl.: C12Q 1/06, C12Q 1/04

(54) **Verfahren zur Quantifizierung von Methangas-Bakterien**
Method of quantifying methane gas bacteria
Méthode pour mesurer la quantité de bactéries productrices de méthane

(30) Priorität: 31.03.1988 DE 3811098
(43) Veröffentlichungstag der Anmeldung: 18.10.1989
(73) Patentinhaber: Orpegen Medizinisch-Molekularbiologische Forschungsgesellschaft m.b.H., 69115 Heidelberg (DE)
(72) Erfinder: Nader, Werner, Dr., D-6900 Heidelberg (DE); Nebe, Gerhard, D-6802 Ladenburg (DE); Nebe, Carl Thomas, Dr. med., D-6802 Ladenburg (DE); Birr, Christian, Prof. Dr., D-6900 Heidelberg (DE)
(74) Vertreter: Huber, Bernhard, Dipl.-Chem.

(56) Entgegenhaltungen:
- GB-A- 2 155 631
- APPLIED AND ENVIRONMENTAL MICROBIOLOGY, Band 49, Nr. 5, Mai 1985, American Society for Microbiology, Washington DC (US); J. DOLFING et al., Seiten 1142-1145#
- JOURNAL OF BIOTECHNOLOGY, Band 4, 1986, Elsevier Science Publishers B.V., Amsterdam (NL); B.W. REUTER et al., Seiten 325-332#
- B.D. Davis et al., MICROBIOLOGY, Ausgabe 3, 1984, Kapitel 3: "Energy production", and Kapitel 5: "Bacterial nutrition and growth", Harper & Row, New York, NY (US); Seiten 31-44, 59-70#
- CHEMICAL ABSTRACTS, Band 103, 1985, Columbus, OH (US); Seite 389, Nr. 157062q#

## Beschreibung

Die Erfindung betrifft ein Verfahren zur Quantifizierung von Methangas-Bakterien.

Organisches Material wird unter Luftabschluß und bei Anwesenheit einer geeigneten Mikroflora zu Methangas umgewandelt. Dabei wird das Material in einem mehrstufigen Abbau zuerst hydrolysiert (Hydrolyse-Phase), dann zu organischen Säuren vergoren (Versäuerungs-Phase), durch acetogene Bakterien zu molekularem Wasserstoff und Kohlendioxid umgewandelt (acetogene Phase) und dann durch methanogene Bakterien (Methangas-Bakterien) zu Methangas umgewandelt (methanogene Phase). Diese Reaktionskette kann nur dann effizient ablaufen, wenn sich in der Bakterienmischpopulation genügend Methangas-Bakterien befinden und vermehren können. Andernfalls wird durch die einzelnen Zwischenprodukte der gesamte Abbauprozeß gehemmt.

Dieser Abbauweg findet in der Natur überall dort statt, wo organisches Material unter Luftabschluß abgebaut wird, also in Sümpfen und Teichen, in eutrophierten Gewässern, auf dem Grund der Ozeane und in den Verdauungstrakten von Mensch und Tier. In den sogenannten Biogasreaktoren (Fermentoren) wird er dazu genutzt, organischen Abfall aus der Abwasserklärung, der industriellen und der landwirtschaftlichen Produktion in nutzbares Methangas umzuwandeln.

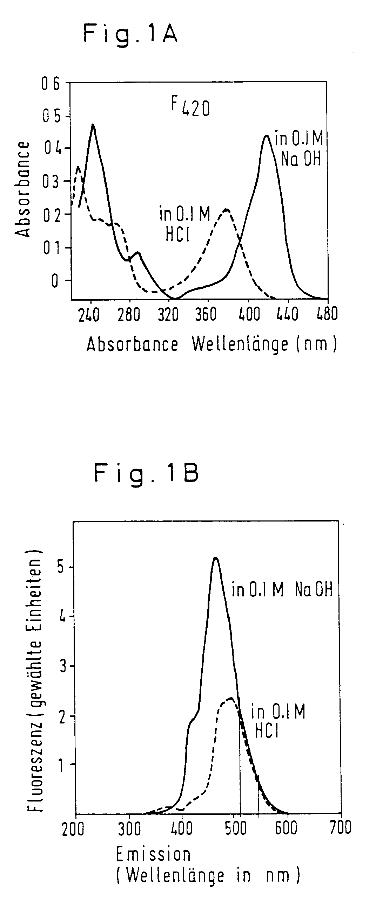

Es ist bekannt, daß Methangas-Bakterien bei Kurzzeitbestrahlung eine Autofluoreszenz aufweisen. Diese Fluoreszenz wird durch den Faktor F₄₂₀ (Flavin F₄₂₀) hervorgerufen, einem Flavin-Mononukleotid-Analog (vgl. S.M. Stronach et al, Anaerobic Digestion Processes in Industrial Waste Water Treatment, Springer-Verlag, Berlin, 1986). Das charakteristische Lichtabsorptions-und Fluoreszenzspektrum dieser Substanz (vgl. Figur 1a und 1b) ermöglicht es, diese Bakterien unter dem Fluoreszenzmikroskop nachzuweisen; eine mikroskopische Quantifizierung oder Auszählen in einer Zählkammer ist aber nicht möglich, da der Farbstoff durch die Lichtabsorption innerhalb von Sekunden zerstört wird und die Bakterien ihre Fluoreszenz verlieren.

Die Konzentrationsbestimmung von F₄₂₀ in Faulschlämmen zur Bestimmung der methanogenen Aktivität ist bekannt; das Verfahren setzt aber voraus, daß das F₄₂₀ aus dem Faulschlamm extrahiert, vorgereinigt und dann fluorimetrisch vermessen wird. B.W. Reuter et al (J. Biotechn. 4 (1986) 325-332) beschreiben die in vivo Messung der F₄₂₀-Fluoreszenz in Kulturen von Methanobakteriumthermoautotrofikum mittels Laser-Spektroskopie ohne vorausgehende zeitaufwendige Extraktionsverfahren; diese Messungen sind aber wegen ihrer Störanfälligkeit auf Reinkulturen von methanogenen Bakterien beschränkt und ermöglichen keine Quantifizierung dieser Bakterien in den in Anspruch 3 beschriebenen Anlagen. Zur Beziehung Methanbildung und F₄₂₀-Gehalt vgl. auch J. Dolfing und J.-W. Mulder, Appl. Environ. Microbiol. 49 (1985) 1142-1145. Aufgabe der vorliegenden Erfindung ist es, eine rasche, sichere und einfache Methode zur Quantifizierung von Methangas produzierenden Bakterien bei anaeroben Abbauprozessen bereitzustellen, die sich insbesondere auch gut zur Kontrolle und Steuerung der Methangasbildungskapazität von Biogasreaktoren eignet. Diese Aufgabe wird mit dem erfindungsgemäßen Verfahren gelöst.

Gegenstand der Erfindung ist ein Verfahren zur Quantifizierung von Methangas-Bakterien, insbesondere zur Kontrolle der Methangasbildungskapazität von Reaktoren mit methanogenen Bakterien, das dadurch gekennzeichnet ist, daß man eine repräsentative Probe eines die Methangas-Bakterien enthaltenden Mediums während höchstens einer Sekunde einer Kurzzeitbestrahlung von Licht der Wellenlänge 395 bis 440 nm aussetzt und die hierdurch angeregte Fluoreszenz durchflußzytometrisch bestimmt.

Für eine gut brauchbare Quantifizierung und zur Bestimmung des prozentualen Anteils von Methangas-Bakterien an den Gesamtbakterien ist es zweckmäßig, neben den Methangas-Bakterien auch die Gesamtmenge der Mikroorganismen mitzubestimmen. Dies kann gleichzeitig geschehen durch Messung der Lichtstreuung, die allerdings außer den Mikroorganismen auch andere feine Partikel erfaßt, oder durch Anfärbung der DNS der Mikroorganismen, wobei dann die beiden Farbsignale gleichzeitig bestimmt werden. Streulichtbestimmung und Anfärbung über die DNS können auch parallel durchgeführt werden, wodurch sich die Genauigkeit weiter steigern läßt.

In einer zweckmäßigen Ausführungsform des erfindungsgemäßen Verfahrens wird deshalb auch die Gesamtmenge der vorhandenen Mikroorganismen durch Streulichtmessung und/oder Anfärbung der DNS bestimmt.

Zur Vorbereitung für die Durchflußzytometrie sind die Proben von störenden Begleitstoffen zu befreien und gegebenenfalls auf geeignete Weise vorzubehandeln. Dies kann auf eine hierfür allgemein bekannte Weise erfolgen; in der Regel genügt eine Filtration; so können zum Beispiel Faulschlammproben aus den Faultürmen einer konventionell gebauten Biogasanlage für die Reinigung kommunaler Abwässer über Papierfilter abfiltriert werden und das Filtrat ohne weitere Aufarbeitung im Zytometer vermessen werden.

Die Durchflußzytometrie (Laser-Durchflußzytometrie; Flow Cytometry FCM) ist eine allgemein bekannte und häufig verwendete Methode zur Analyse von Zellen jeglicher Art, wobei mehrere Parameter, wie DNA, RNA und Proteingehalt, Immunofluoreszenz, Zellgröße und Zellform gleichzeitig vermessen werden können (vgl. z.B. Bio / Technology, 3 (1985) 337-356; Firmenprospekt "Zytometrie" der Firma Orpegen, Heidelberg;Firmenprospekt der Skatron A/S, N-3401 Lier). Die Auswahl der für das erfindungsgemäße Verfahren verwendeten verfahrensmäßigen und apparativen Ausführungsformen richtet sich deshalb insbesondere nach der spezifischen zu untersuchenden Probe, nach der Probevorbereitung und nach den Verfahrensmaßnahmen. Zur gleichzeitigen Messung der Lichtstreuung und/oder Anfärbung der DNS eignet sich zum Beispiel sehr gut ein Skatron-Durchflußzytometer. In der europäischen Patentanmeldung 89 105 555.0 (EP-A-336 281) wird die Durchflußzytometrie zur Überwachung von im Belebtschlamm einer Kläranlage häufig auftretenden Mikroorganismen verwendet; soweit sich die Verfahrensmaßnahmen und apparative Ausgestaltungen dieses Verfahrens auf das Verfahren der vorliegenden Anmeldung übertragen lassen, sind sie Bestandteil dieser Beschreibung.

In einer Ausführungsform des erfindungsgemäßen Verfahrens werden die Bakterien in einer Suspension durch ein blaues Anregungslicht bestrahlt, das von einer Quecksilberdampflampe ausgeht und im Bereich von 395 bis 440 nm gefiltert ist. Simultan werden durch zwei Photomultiplier Streulicht und von den Bakterien ausgehendes Fluoreszenzlicht, das im Bereich von 470 nm gefiltert ist, gemessen und diese Signale durch einen Computer analysiert. Die Bakterien werden nur für Sekundenbruchteile dem Anregungslicht ausgesetzt, so daß die Photolyse des F₄₂₀ ausgeschlossen ist. Diese Meßanordnung ermöglicht es, sämtliche Bakterien über das Streulicht zu vermessen und die Methangas-Bakterien über das zusätzliche Fluoreszenzlicht quantitativ zu erfassen.

Mit dem erfindungsgemäßen Verfahren ist es also möglich, eine genaue, rasche und einfache Quantifizierung von Methangas-Bakterien über ihre Eigenfluoreszenz in situ, z.B. in Faulschlämmen, vorzunehmen, also ohne die mit den bisher bekannten Verfahren verbundenen Nachteile, wie z.B. der Extraktion. Insbesondere ist es mit dem erfindungsgemäßen Verfahren möglich, auf schnelle und einfache Art das Abbaupotential von Faulgemischen im allgemeinen und die Leistungsfähigkeit von Reaktoren mit methanogenen Bakterien im besonderen zu beurteilen. Es ist weiterhin möglich, durch eine vollautomatische und on-line geschaltete Meßstation die Biogasanlage kontinuierlich zu kontrollieren und zu steuern. Gegenstand der Erfindung ist deshalb auch die Verwendung des erfindungsgemäßen Verfahrens zur Kontrolle und Steuerung von Reaktoren mit methanogenen Bakterien, die über die Konzentration von Methangas-Bakterien erfolgt. Eine solche Kontrolle und Steuerung läßt sich auch vollautomatisch durchführen. Daher betrifft die Erfindung insbesondere auch die Verwendung des erfindungsgemäßen Verfahrens zur automatischen Steuerung von Anlagen mit Methangasbakterien, bei der in einer auf der Durchflußzytometrie beruhenden Meßwerterfassungsstelle die Konzentration der Methangasbakterien bestimmt und als Regelgröße für die Steuerung der Anlage, z.B. über Regelung von Pumpen oder Heizelementen, genutzt wird. Unter dem Begriff "Reaktoren mit methanogenen Bakterien" werden im Rahmen der Erfindung sämtliche Anlagen verstanden, die mit methanogenen Bakterien betrieben werden. Hierunter fallen z.B. sämtliche Biogasreaktoren, Fermenter, Faulbehälter oder Faultürme, die zur Beseitigung festen oder flüssigen Abfalls dienen oder die zur Herstellung von Methangas ausgelegt sind.

Daneben eignet sich das erfindungsgemäße Verfahren aber zum Beispiel auch zur qualitativen und quantitativen Bestimmung (Quantifizierung) von Methangas-Bakterien und deren prozentualem Anteil in Proben aus dem tierischen und menschlichen Verdauungstrakt, wodurch es eine wertvolle veterinär- und humanmedizinische Untersuchungsmethode für diagnostische und auch therapeutische Zwecke darstellt, und zur Kontrolle und Beurteilung von natürlich auftretenden Faulungsprozessen von Ökosystemen, wie z.B. des Eutrophierungsgrades eines Gewässers.

Weiterer Gegenstand der Erfindung ist deshalb auch die Verwendung des erfindungsgemäßen Verfahrens zur Bestimmung von Methangas-Bakterien im tierischen und menschlichen Verdauungstrakt und zur Kontrolle und Beurteilung von natürlich auftretenden Faulungsprozessen von Ökosystemen.

Die nachfolgenden Beispiele sollen die Erfindung nun näher erläutern, ohne sie darauf zu beschränken.

### Beispiel 1

### Messung einer Reinkultur von Methangas-Bakterien

Methanogene Bakterien der Spezies Methanococcus vinelandii wurden in Reinkultur bis zu einer Dichte von 2,7 x 10⁸ pro ml angezogen und dann im Durchflußzytometer vermessen.

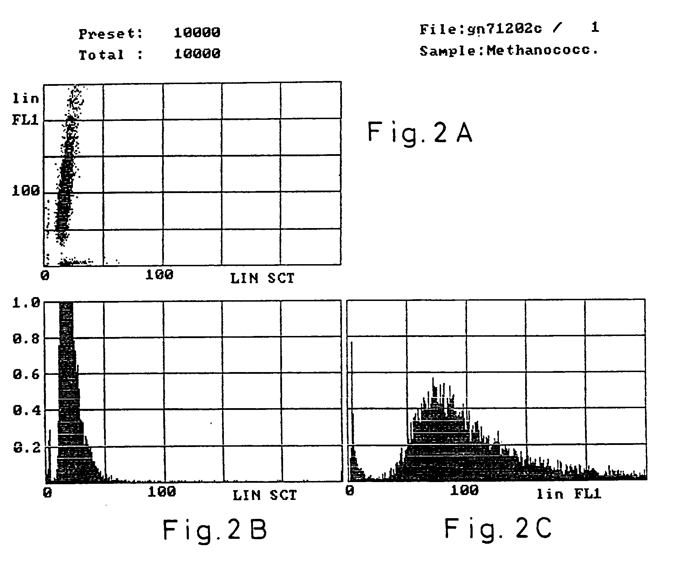

Die Figur 2A, B und C zeigt die Vermessung einer Reinkultur von Methanococcus vinelandii mit dem Durchflußzytometer.
A: Jeder Punkt entspricht einem gemessen Bakterium; zu jedem Bakterium wurde nach Anregung mit Blaulicht im Bereich von 395 bis 440 nm das Gesamtstreulicht und das 470 nm-Fluoreszenzlicht vermessen.
B: Auftragung des linearen Streulichtanteils gegen die Häufigkeit der Messungen;
C: Auftragung des linearen Fluoreszenzsignals gegen die Häufigkeit der Messungen; LIN SCT bedeutet die lineare Streulichtintensität; LIN FL1 bedeutet die lineare Fluoreszenzlichtintensität.

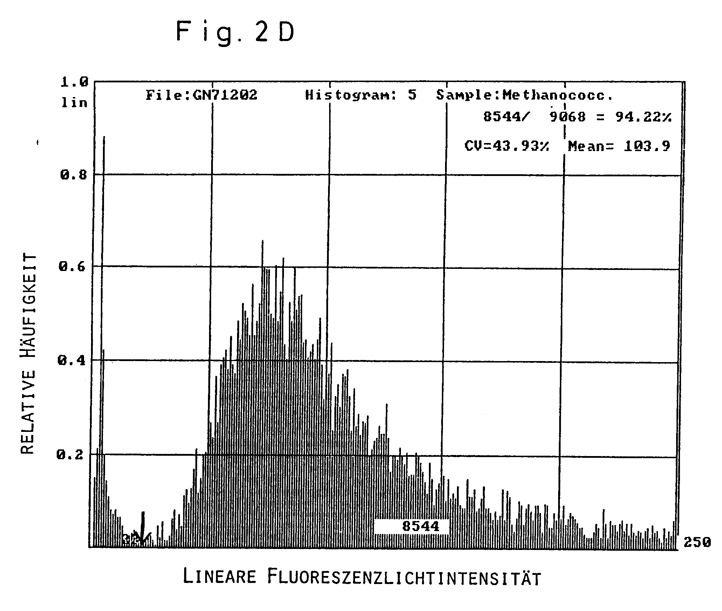

Die Figur 2D zeigt die Auswertung von Figur 2C: der Pfeil markiert die Fluoreszenzintensität, von der an Partikel als fluoreszent eingestuft werden. Daraus ergibt sich ein Anteil von Fluoreszenzbakterien von 94,22 %.

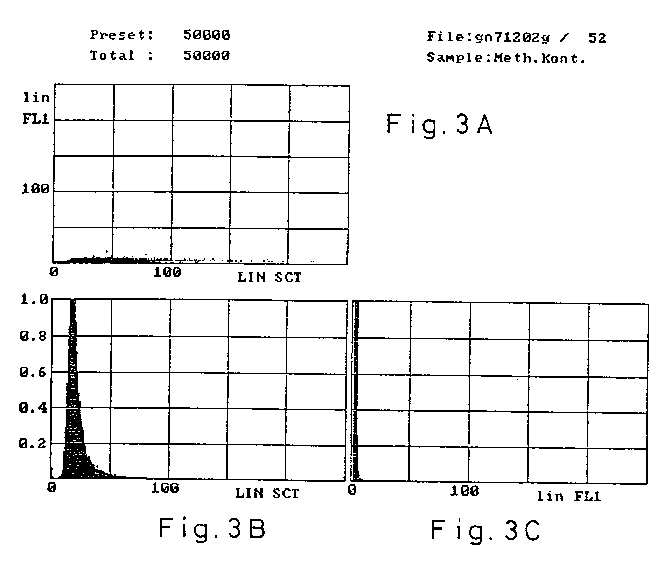

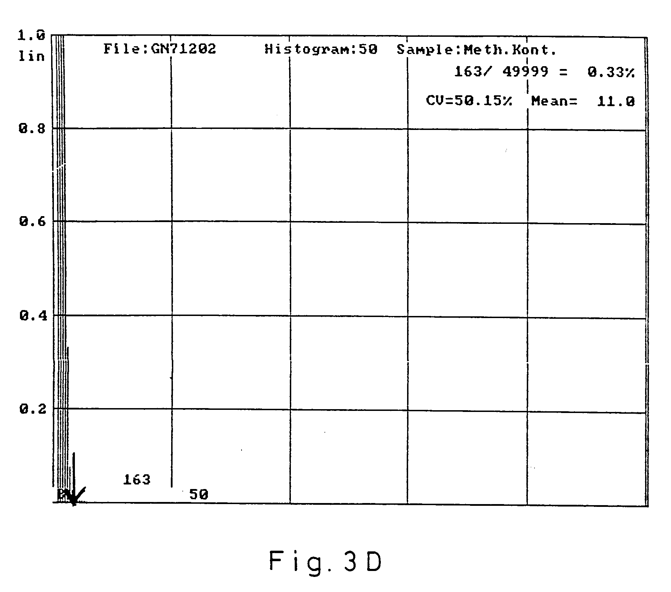

Wie aus der Auswertung des Histogramms der Fluoreszenzintensität gegen die Signalhäufigkeit aus Figur 2D hervorgeht, zeigen also 94 % der 100 000 Streulicht abgebenden Partikel ein deutliches Fluoreszenzsignal. Zur Kontrolle wurde die gleiche Bakteriensuspension eine Stunde im Abstand von 5 cm dem Licht einer 100 W starken Quecksilberdampflampe ausgesetzt, wobei das Flavin photolysiert wird. Diese Suspension wurde erneut im Zytometer vermessen: die Figur 3A, B und C zeigt die Vermessung einer Reinkultur von Bakterien der Art Methanococcus vinelandii nach Photolyse des F₄₂₀ mit Blaulicht; die Figur 3D zeigt die Auswertung von Figur 3C.

Aus der Figur 3 geht hervor, daß die Bakterien das Erregerlicht zwar noch streuen, aber kein Fluoreszenzlicht mehr abgeben.

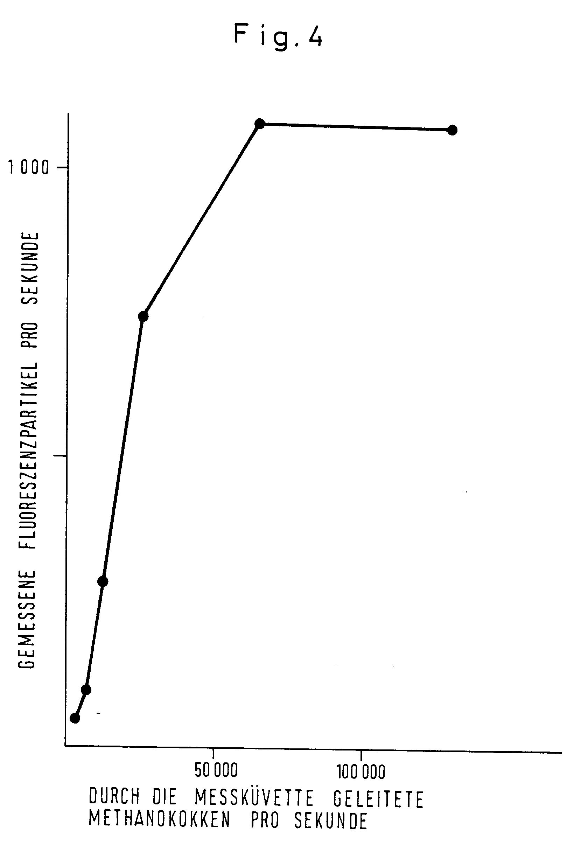

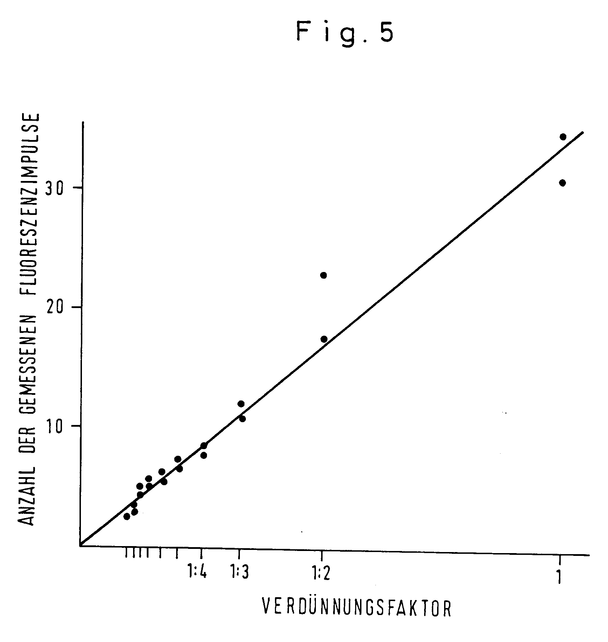

Die Figur 4 zeigt die Abhängigkeit der gemessenen Fluoreszenzimpulse von der Anzahl der am Meßstrahl vorbeigeleiteten Bakterien, und die Figur 5 zeigt die Abhängigkeit der gemessenen Fluoreszenzimpulse von der Verdünnung einer Methanococcus-Reinkultur.

Die Figuren 4 und 5 zeigen, daß die Methangas-Bakterien über das Fluoreszenzlicht in einem breiten Verdünnungs-und Probendosierungsbereich quantitativ von der Meßanordnung erfaßt werden können. Nur ab einer durch den Meßstrahl geleiteten Bakterienzahl von 6,8 x 10⁴/Sekunde erfaßt die Meßanordnung nicht mehr alle Methangas-Bakterien.

### Beispiel 2

### Quantifizierung von Methangas-Bakterien im Faulschlamm einer Biogasanlage

Faulschlammproben aus den Faultürmen einer konventionell gebauten Biogasanlage für die Reinigung kommunaler Abwässer wurden über Papierfilter abfiltriert und das Filtrat ohne weitere Aufarbeitung im Zytometer vermessen.

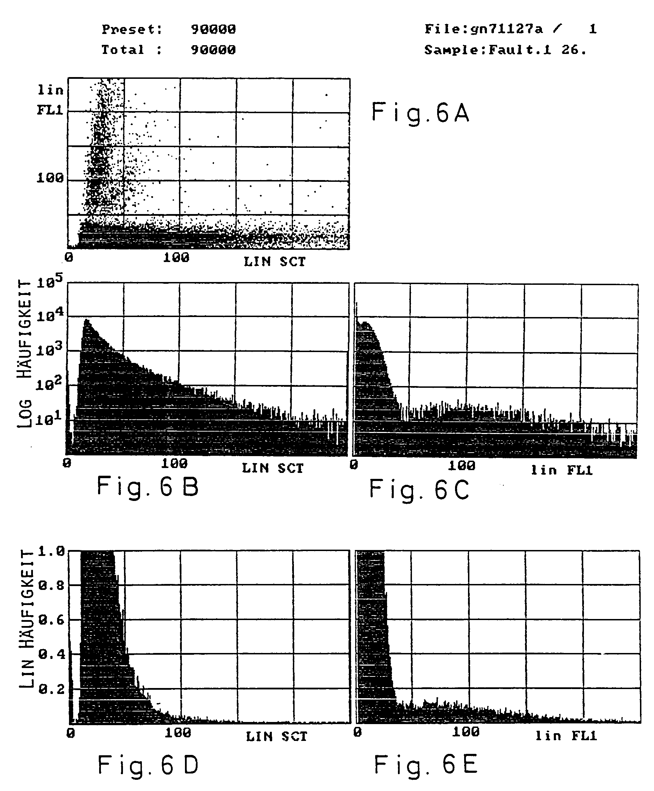

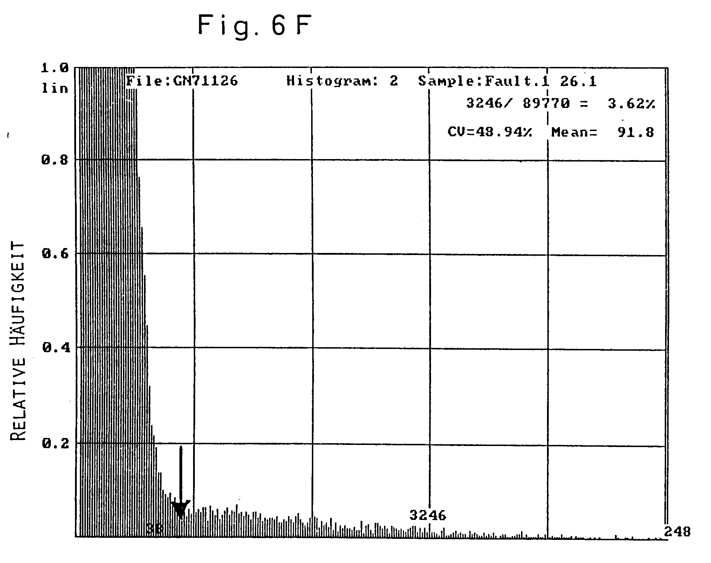

Die Figur 6 zeigt die Vermessung einer gefilterten Faulschlammprobe aus einem Faulturm der Biogasanlage; LIN SCT bedeutet die lineare Streulichtintensität und LIN FL1 die lineare Fluoreszenzlichtintensität. Die Figur 6F zeigt die Auswertung der Figur 6E.

Wie aus der Figur 6 hervorgeht, lassen sich die Methangas-Bakterien durch ihre spezifische Fluoreszenz von den anderen Bakterien, die nur Streulicht produzieren, unterscheiden. Dabei kann sowohl ihr prozentualer Anteil an der Bakteriengesamtpopulation als auch ihre absolute Zelldichte in der Suspension bestimmt werden. Die Auswertung erfolgt durch ein Histogramm der Zellhäufigkeit gegen die Fluoreszenzintensität, wobei sich die Methangas-Bakterien sowohl in der linearen als auch in der halblogarithmischen Auftragung deutlich von den anderen Bakterien absetzen. Zur Kontrolle wurde die Probe wie in Beispiel 1 durch das Licht einer Quecksilberdampflampe ausgebleicht und dann erneut vermessen; in dieser Probe sind nur noch Partikel mit Streulicht, aber ohne Fluoreszenzlichtanteil zu messen.

Die gleiche Probe wurde nun zehnmal hintereinander vermessen und der prozentuale Anteil von Methangas-Bakterien an den Gesamtbakterien bei jeder Einzelmessung bestimmt. Es ergab sich ein Durchschnittswert von 3,71% mit einer Standardabweichung von 0,186.

Eine Kontrolle und Steuerung von Reaktoren mit methanogenen Bakterien über das erfindungsgemäße Verfahren kann z.B. auf die folgende Weise erfolgen:

Es wird erfindungsgemäß die Konzentration der Methangas-Bakterien bestimmt, und durch die folgenden Maßnahmen kann ihre Konzentration beeinflußt (konstant gehalten) und damit eine Optimierung der Vergärung erreicht werden:
1) Optimierung der physikochemischen Parameter des Reaktors (pH-Wert über Kalkzugabe, Temperatur über Reaktorheizung, Durchmischung des Faulschlammgemisches über die entsprechenden Umwälzpumpen);
2) Abstimmung des Mischverhältnisses Impfschlamm zu eingebrachtem Abfall auf eine optimale Konzentration der Methangas-Bakterien; und/oder
3) Zudosierung von Aktivatoren (z.B. kommerziell erhältlicher Polyelektrolyte (z.B. Bio-Klar Algin, Methan-aktiv)) oder von Trockenbakterien der hydrolytischen und säurebildenden Stufe der Faulung.

Die Steuerung und Kontrolle von Anlagen mit Methangas-Bakterien ist auch vollautomatisch möglich, wobei die Konzentration der methanogenen Bakterien in einer auf der Durchflußzytometrie beruhenden Meßwerterfassungsstelle bestimmt und als Regelgröße zur Steuerung von Pumpen (z.B. zur Zudosierung von Bakterien, Impfschlamm, Substrat oder Aktivatoren) oder Heizelemente (zur Optimierung der Reaktortemperatur) benutzt wird.

## Patentansprüche

1. Verfahren zur Quantifizierung von Methangas-Bakterien, insbesondere zur Kontrolle von Methangasbildungskapazität von Reaktoren mit methanogenen Bakterien, **dadurch gekennzeichnet,** daß man eine repräsentative Probe eines die Methangas-Bakterien enthaltenden Mediums während höchstens einer Sekunde einer Kurzzeitbestrahlung von Licht der Wellenlänge 395 bis 440 nm aussetzt und die hierdurch angeregte Fluoreszenz durchflußzytometrisch bestimmt.

2. Verfahren nach Anspruch 1,
**dadurch gekennzeichnet,** daß man gleichzeitig die Gesamtmenge der vorhandenen Mikroorganismen durch Streulichtmessung und/oder Anfärbung der DNS bestimmt.

3. Verwendung des Verfahrens nach einem der Ansprüche 1 oder 2 zur Kontrolle und Steuerung von Reaktoren mit methanogenen Bakterien.

4. Verwendung des Verfahrens nach einem der Ansprüche 1 oder 2 zur Bestimmung von Methangas-Bakterien in einer dem tierischen oder menschlichen Verdauungstrakt entnommenen Probe.

5. Verwendung des Verfahrens nach einem der Ansprüche 1 oder 2 zur Kontrolle und Beurteilung von natürlich auftretenden Faulungsprozessen und Ökosystemen.

## Claims

1. Process for the quantification of methane gas bacteria, especially for the monitoring of the methane gas formation capacity of reactors with methanogenic bacteria, characterised in that one subjects a representative sample of a medium containing the methane gas bacteria for at most one second to a brief irradiation by light of the wavelength 395 to 440 nm and determines the fluorescence hereby excited by means of flow cytometry.

2. Process according to claim 1, characterised in that one simultaneously determines the total amount of micro-organisms present by scattered light measurement and/or staining of the DNA.

3. Use of the process according to one of claims 1 or 2 for the monitoring and control of reactors with methanogenic bacteria.

4. Use of the process according to one of claims 1 or 2 for the determination of methane gas bacteria in a sample taken from the animal or human digestive tract.

5. Use of the process according to one of claims 1 or 2 for the monitoring and assessment of naturally-occurring putrefaction processes and ecosystems.

## Revendications

1. Procédé de détermination quantitative des bactéries productrices de méthane, en particulier pour le contrôle de la capacité de formation de méthane des réacteurs à bactéries méthanogènes, caractérisé en ce que l'on expose pendant une seconde au plus un échantillon représentatif d'un milieu contenant les bactéries productrices de méthane à une irradiation de courte durée par une lumière d'une longueur d'onde de 395 à 440 nm et l'on détermine par cytométrie en flux la fluorescence ainsi provoquée.

2. Procédé selon la revendication 1, caractérisé en ce que l'on détermine en même temps la quantité totale des micro-organismes présents par mesure de la lumière diffusée et/ou par coloration de l'ADN.

3. Utilisation du procédé selon l'une des revendications 1 ou 2 pour le contrôle et la commande de réacteurs à bactéries méthanogènes.

4. Utilisation du procédé selon l'une des revendications 1 ou 2 pour la détermination des bactéries productrices de méthane dans un échantillon prélevé dans les voies digestives animales ou humaines.

5. Utilisation du procédé selon l'une des revendications 1 ou 2 pour le contrôle et l'évaluation des processus de putréfaction naturels et des écosystèmes.
